(19) Europäisches Patentamt
European Patent Office
Office européen des brevets

(11) **EP 3 699 265 A1**

(12) **EUROPEAN PATENT APPLICATION**
published in accordance with Art. 153(4) EPC

(43) Date of publication:
**26.08.2020 Bulletin 2020/35**

(21) Application number: **18869237.0**

(22) Date of filing: **17.10.2018**

(51) Int Cl.:
*C12N 5/0735* (2010.01)  *C12N 5/077* (2010.01)
*C12N 5/10* (2006.01)

(86) International application number:
**PCT/JP2018/038729**

(87) International publication number:
**WO 2019/078278 (25.04.2019 Gazette 2019/17)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **18.10.2017  JP 2017202029**

(71) Applicant: **Kyoto University
Kyoto-shi, Kyoto 606-8501 (JP)**

(72) Inventors:
• **LIU, Li
Kyoto-shi
Kyoto 606-8501 (JP)**
• **YU, Leqian
Kyoto-shi
Kyoto 606-8501 (JP)**
• **LI, Junjun
Kyoto-shi
Kyoto 606-8501 (JP)**

(74) Representative: **Hoffmann Eitle
Patent- und Rechtsanwälte PartmbB
Arabellastraße 30
81925 München (DE)**

(54) **METHOD FOR PRODUCING PLURIPOTENT STEM CELLS TO BE DIFFERENTIATED INTO CARDIOMYOCYTES**

(57) The disclosure provides a method of preparing a pluripotent stem cell suitable for differentiating into a cardiomyocyte comprising (1) culturing at least one pluripotent stem cell on a low cell adhesion substrate to generate a dome-like colony; and (2) collecting a cell from the dome-like colony. The disclosure also provides a method of preparing a population of cardiomyocytes comprising inducing myocardial differentiation of a population of pluripotent stem cells prepared by the method described above.

Fig. 1

## Description

### TECHNICAL FIELD

This application claims the benefit of priority of Japanese Patent Application No. 2017-202029, the entire contents of which are incorporated herein by reference.

The disclosure relates to a method of preparing a pluripotent stem cell suitable for differentiating into a cardiomyocyte, a method of preparing a cardiomyocyte from the pluripotent stem cell, and the pluripotent stem cell prepared by the former method or the cardiomyocyte prepared by the latter method.

### BACKGROUND

Pluripotent stem cells, e.g., embryonic stem cells (ES cells) or induced pluripotent stem cells (iPS cells), have been studied enthusiastically and seem useful in various fields, such as regenerative therapy, drug development and drug safety evaluation. For example, a desired scenario is inducing differentiation of human pluripotent stem cells, e.g., iPS cells, into cardiomyocytes, culturing the cardiomyocytes to give a myocardial tissue sheet, and using the sheet for transplantation, drug toxicity assessment, or pharmacokinetics assessment.

For such purpose, homogeneous pluripotent stem cells having high pluripotency and differentiation potential are required. However, pluripotency and differentiation potential of ES cell lines has been reported to differ between cell lines (Non-Patent Literatures 1-3). A population of iPS cells is usually a mixture of heterogeneous cells having different properties, since iPS cells are generated by initializing a large number of cells obtained from a body by transfection of the reprogramming genes. Properties of established iPS cell lines are different from each other.

The efficiencies of differentiation of iPS cells induced by existing methods vary depending on the type of target cells. For example, when the target cells are liver cells or alveolar epithelial cells, the efficiency is low and the function of the differentiated cells is insufficient. On the other hand, highly developed methods are available for inducing myocardial differentiation (Patent Literatures 1 and 2, Non-Patent Literatures 4 and 5), and the obtained populations of cardiomyocytes have almost no problem in their homogeneity. However, cardiomyocytes derived from pluripotent stem cells, unlike cardiomyocytes in a living body, are not fully matured, and thus have not been used for drug assessment or regeneration therapy yet.

Methods for obtaining differentiated cells, particularly differentiated cardiomyocytes, from pluripotent stem cells are still in development. One proposed technique for improving such methods is use of a nanofiber having a fiber diameter of nanometer order as a substrate on which pluripotent stem cells are cultured (Patent Literature 3 and Non-Patent Literature 6).

### REFERENCES

### PATENT LITERATURE

[Patent Literature 1] WO2013/111875
[Patent Literature 2] WO2015/182765
[Patent Literature 3] JP 6024047 B

### NON-PATENT LITERATURE

[Non-Patent Literature 1] Osafune, K. et al., Nat. Biotechnol. 26, 2007-2009 (2008).
[Non-Patent Literature 2] Sung-Eun Kim et. al., Comparative analysis of the developmental competence of three human embryonic stem cell lines in vitro.
[Non-Patent Literature 3] The International Stem Cell Initiative, Nat. Biotechnol. 25, 803-816 (2007)
[Non-Patent Literature 4] Minami I, et al., Cell Rep. 2012 Nov 29; 2(5) : 1448-60
[Non-Patent Literature 5] Lian, X. et al. Nat. Protoc. 8, 162-175 (2013)
[Non-Patent Literature 6] L. Liu et al., Biomaterials 35 (2014) 6259-67

SUMMARY

An object of the disclosure is to provide a method of preparing a pluripotent stem cell suitable for differentiating into a cardiomyocyte. Another object of the disclosure is to provide a method of preparing a cardiomyocyte from the pluripotent stem cell. A further object of the disclosure is to provide the pluripotent stem cell prepared by the former method or the cardiomyocyte prepared by the latter method.

The inventors have found that pluripotent stem cells cultured on a low cell adhesion substrate form two types of colonies, monolayer colonies and dome-like colonies. As demonstrated in the Examples below, the cells constituting dome-like colonies have higher myocardial differentiation potential than the cells constituting monolayer colonies.

Accordingly, an aspect of the disclosure provides a method of preparing a pluripotent stem cell suitable for differentiating into a cardiomyocyte comprising

(1) culturing at least one pluripotent stem cell on a low cell adhesion substrate to generate a dome-like colony; and
(2) collecting a cell from the dome-like colony.

Another aspect of the disclosure provides a composition for preparing a cardiomyocyte comprising the pluripotent stem cell prepared by the method above.

Another aspect of the disclosure provides a method of preparing a population of cardiomyocytes comprising inducing myocardial differentiation of a population of pluripotent stem cells prepared by the method above.

Another aspect of the disclosure provides a population of cardiomyocytes prepared by the method above.

According to the disclosure, a method of preparing a pluripotent stem cell suitable for differentiating into a cardiomyocyte is provided. A highly matured cardiomyocyte can be obtained by inducing myocardial differentiation of the pluripotent stem cell.

BRIEF DESCRIPTION OF DRAWINGS

Fig. 1: A schematic representation of single-cell dissociation and culture strategy.

Fig. 2: Single human pluripotent stem cells (hPSCs) grew into multicellular clones during the culture course from Day 1 to Day 12. These clones demonstrated two types of morphologies: flat-monolayer clones (top panel) and domed-multilayer clones (bottom panel).

Fig. 3: Left panel: SEM images showing the morphological difference between monolayer colony on gelatin (MCoG) cells and dome-like colony on gelatin (DCoG) cells. Right panel: Cross-section images of MCoG and DCoG colonies observed by optical coherence tomography (OCT) microscopy system in real time.

Fig. 4: Heights of MCoG and DCoG colonies on culture day 1, 2, 3 and 4 (mean $\pm$ SD, n=10). The heights were measured by OCT microscopy system.

Fig. 5: Top panel: Overview of the myocardial differentiation protocol. Bottom panel: Phase contrast images of MCoG and DCoG cells during differentiation on day 0, 1, 3, 5, and 10. Scale bars: 150 $\mu$m.

Fig. 6: Immunofluorescence images of DCoG and MCoG cells on day 12 of myocardial differentiation. Top-left and bottom-left: nuclei, Top-right: cTnT, Bottom-right: Actinin. Scale bars: 100 $\mu$m.

Fig. 7: Left: Representative strain of cardiomyocytes derived from DCoG and MCoG cells. The cardiomyocytes derived from DCoG cells showed higher strain rate at every beat. Right: Strain rate per beat (mean $\pm$ SEM, n = 4, *P < 0.05).

Fig. 8: Heatmap of qPCR with 18 cardiac related genes: undifferentiated (day 0) and differentiated (day 10) DCoG and MCoG cells. The cardiac related genes, *ACTN2, DES, MYH7, MYL2, MYL3, MYL7, TNNI3,* and *TNNT2,* are related to cardiomyocyte structure; *GATA4, HAND2,* and *HKX2-5* are cardiomyocyte related transcription factors; *NPPA* and *RYR2* are cardiomyocyte receptors; *KCNQ1, PLN,* and *SLC8A1* are related to cardiomyocyte ion channels; *CKM* is a cardiomyocyte enzyme; and *MB* is a cardiomyocyte transporter.

Fig. 9: Morphology change of DCoG and MCoG cells on different substrates during long-term passage. In each condition, the left panel is the phase contrast image and the right panel is the SEM image.

Fig. 10: SEM images of single MCoG and DCoG cells on different substrates. The DCoG cell does not spread on the gelatin nanofibrous (GNF) substrate. Arrows indicate cells engaged in spreading.

Fig. 11: Adhesion rate of MCoG and DCoG cells on the GNF substrate. Top panel: Schematic diagram of the adhesion test. The number of adhered cells was determined at 10, 20, and 40 min after cell seeding. The cell adhesion rate was calculated as follows: Cell adhesion rate (%) = (number of adhered cells/number of seeded cells) x 100%. Statistical analysis was performed using t-tests. Results from three independent experiments are shown. Bottom panel: MCoG cells showed a higher adhesion rate than DCoG cells (mean $\pm$ SD, n = 3, *P < 0.05).

Fig. 12: Quantification of cell-substrate adhesion using a shock wave-based method. Fraction of detached cells was plotted as a function of hydrodynamic pressure P. Data points were fitted with the cumulative distribution function of normal distribution, and the critical pressure P* was determined as the required pressure at which 50% of cells were detached (mean $\pm$ SE, n $\geq$ 500). Four conditions were investigated: MCoG cells on Matrigel (MG) (MCoG-MG), DCoG cells on MG (DCoG-MG), MCoG cells on GNF (MCoG-GNF), and DCoG cells on GNF (DCoG-GNF).

Fig. 13: Relative expression of pluripotency-associated genes and epiblast genes in MCoG and DCoG cells on MG and GNF substrates (mean $\pm$ SD, n $\geq$ 3, *P< 0.05).

Fig. 14: Immunofluorescence images of F-actin and G-actin in MCoG and DCoG cells on the GNF substrate. Confocal microscopy images show the basal surface.

Fig. 15: Quantification of F-actin and G-actin levels 2 hr after seeding MCoG and DCoG cells. Total integrated fluorescence of phalloidin (anti-F-actin) and DNaseI (anti-G-actin) was normalized to the fluorescence of MCoG cells (mean $\pm$ SD, n = 3, *P< 0.05, **P< 0.01).

Fig. 16: The intensity correlation quotients (ICQ) indicating the colocalization of MAL and the nucleus in MCoG and DCoG cells (mean $\pm$ SD, n = 3, *P< 0.05).

Fig. 17: Relative expression of E-cadherin in MCoG and DCoG cells cultured on GNF substrates on day 3 (mean $\pm$ SD, n=3, *P< 0.05).

Fig. 18: MCoG and DCoG cell adhesion curves on Matrigel-coated substrates with gradient of coating concentrations (0.1 to 40 $\mu$g/cm$^2$) (mean $\pm$ SD, n=4, *P< 0.05, **P< 0.01).

Fig. 19: MCoG and DCoG cell adhesion curves on substrates coated with various hydrogels (FN: fibronectin, VN: vitronectin, and LN: laminin) with gradient of coating concentrations (0.01 to 8 $\mu$g/cm$^2$) (mean $\pm$ SD, n=4).

Fig. 20: Phase contrast images of MCoG and DCoG cells on Matrigel-coated substrates with three different coating concentrations (0.1, 1 and 10 $\mu$g/cm$^2$) (recommended coating concentration for MG: >10 $\mu$g/cm$^2$). Scale bars: 150$\mu$m.

Fig. 21: Relative expression of *KLF4, KLF5,* and *NANOG* in MCoG and DCoG cells on Matrigel-coated substrates with three different coating concentrations (0.1, 1 and 10 $\mu$g/cm$^2$) (mean $\pm$ SD, n=3, *P< 0.05, NS: not significant).

DETAILED DESCRIPTION

Unless otherwise defined, the terms used herein are read as generally understood by a skilled person in the technical fields such as organic chemistry, medical sciences, pharmaceutical sciences, molecular biology, and microbiology. Several terms used herein are defined as described below. The definitions herein take precedence over the general understanding.

When a numerical value is accompanied with the term "about", the value is intended to represent any value in the range of -10% of the value to +10% of the value. For example, "about 20" means "a value from 18 to 22." A range defined with a value of the lower limit and a value of the upper limit covers all values from the lower limit to the upper limit, including the values of both limits. When a range is accompanied with the term "about", both limits are read as accompanied with the term. For example, "about 20 to 30" is read as "18 to 33."

The term "pluripotent stem cell" means a cell having pluripotency and replication competence. The pluripotency means an ability to differentiate into any cells constituting an adult body, and the replication competence means an ability to keep the pluripotency after cell division. The pluripotent stem cell may be newly established or obtained from an established cell line. Examples of the pluripotent stem cells include an embryonic pluripotent stem cell (ES cell), an embryonic germ cell (EG cell), and an induced pluripotent stem cell (iPS cell). The pluripotent stem cell may be of any biological species without limitation, preferably a mammal, more preferably a rodent or primate, and still more preferably a primate. A monkey or human pluripotent stem cell, particularly an ES cell or iPS cell may be used. In one embodiment, the pluripotent stem cell is a human iPS cell.

The term "low cell adhesion substrate" means a substrate on which at least one dome-like colony is formed when pluripotent stem cells are cultured thereon. A low cell adhesion substrate may be obtained by coating the surface of a sheet such as a conventional culture vessel or cover slide with a cell adhesive substance under an appropriate condition.

Cell adhesion of a substrate may be measured by a known method. For example, cell adhesion rate may be determined by seeding a known number of adhesive cells on a substrate, incubating the cells for a predetermined period, e.g., several minutes to several hours, such as 10, 20, 30, or 40 minutes, washing out the cells, counting the cells adhered to the substrate, and calculating the cell adhesion rate according to the following formula;

```
Cell adhesion rate (%) = (number of adhered cells / number
of seeded cells) × 100%.
```

Since the cell adhesion rate depends on the inherent adhesion of the substrate and the inherent adhesion of the cells, this is an indication of the adhesion of the substrate when the adhesion of the cells is constant, for example, the cells are collected from the same tissue or derived from the same cell line.

Alternatively, cell adhesion of the substrate can be measured by the method described in Yoshikawa, H. Y. et al., J. Am. Chem. Soc. 133, 1367-1374 (2011). In this method, an adhesive force between a substrate and a cell population is calculated by giving a shock wave to one point in the cell population adhered to the substrate with a laser pulse and determining the area of detached cells around the point. Cell adhesion can be compared between different substrates by measuring the adhesive forces using cells having a constant adhesion, e.g., cells collected from the same tissue or cells derived from the same cell line.

Any culture vessel generally used for cell culture may be used without limitation. A culture vessel in the form of a petri dish, a plate, a bottle, a chamber, or a multi-well plate, e.g., 6, 12, 24, 48, 96, or 384-well plate, may be used. A sheet, e.g., a cover slide, which is coated with a cell adhesive substance may be placed in a culture vessel. Alternatively, a culture vessel may be produced by coating a sheet, e.g., a cover slide, with a cell adhesive substance and placing another sheet having a hole on the coated sheet. Materials of such culture vessels or sheets are not particularly limited, including inorganic materials, such as metal, glass, and silicone, and organic materials, such as plastic.

Examples of the cell adhesive substances include hydrogels such as Matrigel, fibronectin, vitronectin, and laminin, as well as nanofibers containing a polymer such as gelatin, collagen, polyglycolic acid (PGA), polylactic acid (PLA) and copolymer thereof (PLGA), polycaprolactone (PCL), and polystyrene (PS). These substances are commercially available.

Hydrogels are used at a concentration that provides a substrate with desired low cell adhesion. Hydrogels may be used at a concentration lower than that recommended by the manufacturer. For example, Matrigel, fibronectin, or vitronectin may be used so that it coats the surface of a culture vessel at a concentration in the range of about 0.01 to 1.0 $\mu$g/cm$^2$ or about 0.05 to 0.3 $\mu$g/cm$^2$, e.g., about 0.1 $\mu$g/cm$^2$. For example, laminin may be used so that it coats the surface of a culture vessel at a concentration in the range of about 0.01 to 0.1 $\mu$g/cm$^2$, e.g., about 0.05 $\mu$g/cm$^2$. When other cell adhesive substances are used, the concentration may be adjusted so that the substance provides cell adhesion equivalent to that achieved with any one of the described hydrogels used at a described concentration, for example, Matrigel used at a concentration in the range of about 0.01 to 1.0 $\mu$g/cm$^2$ or about 0.05 to 0.3 $\mu$g/cm$^2$, preferably about 0.1 $\mu$g/cm$^2$. In one embodiment, a low cell adhesion substrate is a substrate that has cell adhesion equivalent to the glass surface coated with Matrigel at a concentration in the range of about 0.01 to 1.0 $\mu$g/cm$^2$ or about 0.05 to 0.3 $\mu$g/cm$^2$, preferably about 0.1 $\mu$g/cm$^2$.

Examples of the polymers constituting the nanofibers include gelatin, collagen, PGA, PLA, PLGA, PCL, and PS. Gelatin may be obtained from any source without limitation, e.g., bovine bone, bovine skin, and pig skin, as well as fish skin or scales, e.g., salmon skin or scales. Methods for extracting and/or purifying gelatin from such sources are well known. Commercially available gelatin may also be used. Collagen may be extracted during gelatin production from any native collagen source before denaturation with an acid or alkaline. The collagen source is not particularly limited. Commercially available collagen, e.g., those marketed as coating substrates for cell culture, may also be used. Non-biopolymers such as PGA, PLA, PLGA, PCL, and PS may be synthesized according to methods well known to those skilled in the art or commercially available. The molecular weight of the polymer is not particularly limited. For example, the appropriate molecular weight of gelatin may be about 10 kDa or more, preferably in the range of about 20 to 70 kDa, and more preferably in the range of about 30 to 40 kDa.

The method of producing a nanofiber from such polymers is not particularly limited, and examples thereof include electrospinning, conjugate melt spinning, and melt blowing. Electrospinning is preferably used. In electrospinning, a polymer is dissolved in an appropriate solvent. The solvent may be an inorganic or organic solvent capable of dissolving the polymer. For example, for producing a gelatin nanofiber a solution of an acid such as acetic acid and formic acid may be preferably used. For producing a collagen nanofiber, for example, 1,1,1,2,2,2-hexafluoro-2-propanol may be used. The concentration of such a polymer solution is not particularly limited. For example, for producing a gelatin nanofiber having a preferable fiber diameter and uniformity, the concentration of gelatin in an acetic acid solution may be in the range of about 5 to 15 w/v%, preferably about 8 to 12 w/v%.

Electrospinning may be carried out according to a known method. In electrospinning a nano-sized fiber is formed by spraying a material with an electric force. A syringe filled with a polymer solution and equipped with a nozzle like an injection needle is connected to a syringe pump capable of driving a fluid flow. A collector on which the nanofiber is collected is placed at an appropriate distance from the nozzle. The collector may be a flat collector or a winding collector. The flat collector may be coated with a substrate such as glass and the nanofiber may be formed on the substrate. The nozzle is connected to the cathode of a power source and the collector is connected to the anode. When a voltage is applied to the syringe pump, the polymer is injected onto the collector to form the nanofiber. The form and diameter of the nanofiber depend on conditions of electrospinning including voltage, distance from the nozzle to the collector, and the inner diameter of the nozzle. Those skilled in the art can appropriately select such conditions to produce a uniform fiber having a desired diameter. For example, the conditions employed in the Examples below or the conditions described in Advanced Drug Delivery Reviews, 61(12): 1084-1096 (2009) or Journal of Cellular and Molecular Medicine, 13(9B): 3475-3484 (2009) may be used.

The nanofiber may have a diameter in the range of about 1 to 1000 nm, preferably about 10 to 800 nm, and more preferably about 50 to 500 nm. The concentration of the polymer on a culture vessel coated with the nanofiber may be in the range of about 0.1 to 10 $\mu$g/cm$^2$, preferably about 1 to 8 $\mu$g/cm$^2$, more preferably about 3 to 5 $\mu$g/cm$^2$.

The nanofiber is preferably cross-linked with an appropriate cross-linker so that the nanofiber has a suitable three-dimensional property and facilitates dissociation of cells at passages. Type of the cross-linker is not particularly limited, and examples of the preferable cross-linkers include water-soluble carbodiimides (WSCs) such as N-ethyl-N'-(dimethylaminopropyl)carbodiimide and N-hydroxysuccinimide (NHS). Two or more cross-linkers may be used in a mixture. The nanofiber may be cross-linked by immersing it in a solution which has a cross-linker dissolved in an appropriate solvent. Those skilled in the art can select suitable conditions such as concentration of the cross-linker and the duration of the immersion in view of the type of the cross-linker.

In one embodiment, a gelatin nanofiber is used. The gelatin nanofiber may be produced as described above, or as described in, e.g., Patent Literature 3 or Non-Patent Literature 6. In one embodiment, the gelatin nanofiber is produced by dissolving gelatin (11 wt%, type B, derived from porcine skin) in a mixture of acetic acid, ethyl acetate, and distilled water (acetic acid: ethyl acetate = 3:2) to prepare a gelatin solution, electrospinning it on a culture cover glass slide (voltage: 11 kV, flow rate: 0.2 mL/h, time: 8 min), and cross-linking it in 0.2 M N-ethyl-N'-(dimethylaminopropyl)carbodiimide and 0.2 M N-hydroxysuccinimide in ethanol for 4 hours. Other nanofibers containing polymers may be produced by adjusting process conditions so that the resulting cell adhesion is equivalent to that achieved by coating a culture vessel with the gelatin nanofiber produced under the conditions described above. In one embodiment, a low cell adhesion substrate is a substrate having cell adhesion equivalent to that of a glass surface coated with a gelatin nanofiber produced under the conditions described above. In one embodiment, a low cell adhesion substrate is a substrate having cell adhesion equivalent to that of a glass surface coated with a gelatin nanofiber at a concentration in the range of about 3 to 5 $\mu$g/cm$^2$.

Pluripotent stem cells may be cultured by a conventional method well known to those skilled in the art. For example, the method described in Patent Literature 3 or Non-Patent Literature 6 may be used. For example, pluripotent stem cells are suspended preferably in a medium containing a ROCK inhibitor, e.g., Y27632, and seeded on a low cell adhesion substrate at a cell density in the range of about $1 \times 10^2$ to $1 \times 10^5$ cells/cm$^2$, preferably about $0.5 \times 10^4$ to $1 \times 10^5$ cells/cm$^2$, more preferably about $2 \times 10^4$ to $6 \times 10^4$ cells/cm$^2$. Preferably, pluripotent stem cells are seeded at a density that allows isolation of a colony derived from one pluripotent stem cell. More preferably, each pluripotent stem cell is singly seeded in a culture vessel or a well of a culture vessel.

Pluripotent stem cells may be cultured in any medium suitable for maintaining them. For example, DMEM/F-12 medium supplemented with 0.1 mM 2-mercaptoethanol, 0.1 mM non-essential amino acids, 2 mM L-glutamic acid, 20% KSR, and 4 ng/ml bFGF, or a synthetic medium such as mTeSR or Stem pro; DMEM, DMEM/F-12, or DME medium containing 10 to 15% FBS, which may optionally contain one or more further components, such as LIF, penicillin/streptomycin, puromycin, L-glutamine, non-essential amino acids, and $\beta$-mercaptoethanol; or a commercially available medium, e.g., a medium for culturing mouse ES cells (TX-WES culture medium; Thromb-X N.V.) or a medium for culturing primate ES cells (Primate ES/iPS Cell Medium; REPROCELL Inc.) may be used. A serum-free medium such as mTeSR medium containing recombinant animal proteins, a xeno-free medium such as TeSR2 medium containing human serum albumin and human bFGF, and a protein-free medium such as E8 medium may also be used. Such medium may contain a ROCK inhibitor, e.g., Y27632.

Pluripotent stem cells may be cultured in a $CO_2$ incubator at a temperature in the range of about 30 to 40 °C, preferably at about 37 °C, and at a $CO_2$ concentration in the range of about 1 to 10%, preferably about 2 to 5%. Preferably, about two days after seeding pluripotent stem cells the medium is changed to a medium containing no ROCK inhibitor, and the medium is changed to a fresh medium every one to two days.

Pluripotent stem cells may be passaged at an appropriate time. The passage may be carried out by dissociating cells from the substrate and reseeding a single cell or a cell population on another substrate. For example, cells can be readily dissociated from the substrate with a dissociation solution containing no enzyme, and cells can be dispersed singly by gentle pipetting. The dissociation solution containing no enzyme may be a dissociation solution conventionally used in methods for mechanically dissociating cells, such as Hanks' solution or a solution containing citric acid and EDTA. Alternatively, cells may be dispersed with an enzyme, such as trypsin, TryoLE(™) Express, collagenase, or dispase. Pluripotent stem cells may be passaged at virtually unlimited number of times.

A colony of pluripotent stem cells is formed by culturing pluripotent stem cells on a low cell adhesion substrate for a suitable period. For example, a single cell proliferates to form a colony in at least about 1 to 2 days and the colony grows as much as possible under the culture conditions. Such colonies have dome-like or flat monolayer morphology that can be clearly distinguished under an optical microscope. The term "dome-like colony" means a colony consisting of plural cell layers, i.e., a multilayer colony. In dome-like colonies cell layers stack vertically, the cells in the lowermost layer adhere to the substrate and the other cells do not. The term "monolayer colony" means a colony essentially consisting of one cell layer. Substantially all of the cells in monolayer colonies adhere to the substrate. The heights of monolayer colonies do not exceed that of one cell, e.g., 50 $\mu$m, whereas the heights of dome-like colonies increase as the colonies grow up. In one embodiment, the dome-like colony has a height of 50 $\mu$m or more, 60 $\mu$m or more, 70 $\mu$m or more, 80 $\mu$m or more, 90 $\mu$m or more, or 100 $\mu$m or more. In one embodiment, the dome-like colony has a height of 50 $\mu$m or more.

In the disclosure a cell that is a constituent of a dome-like colony is referred to as a DCoG cell and a cell that is a constituent of a monolayer colony is referred to as a MCoG cell. As demonstrated in the Examples below, DCoG cells have higher pluripotency and replication competence than MCoG cells. DCoG cells tend to have, e.g., 1.2 times, 1.5 times, 2 times, 2.5 times, 3 times or more, higher expression levels of *NANOG, KLF4,* and *KLF5* than MCoG cells. When cells are individually observed, MCoG cells may flatly spread and form long cell filopodia, whereas DCoG cells may be hemispheric, hardly spread and form few short filopodia.

For collecting cells from a dome-like colony, the whole colony may be collected, or some cells may be collected after the cells in the colony are enzymatically or mechanically separated as described above for the passage of cells. The obtained cells may be frozen and stored by a method known to those skilled in the art. The obtained cells may be cultured on a low cell adhesion substrate or on another substrate suitable for culturing pluripotent stem cells.

The pluripotent stem cells prepared as disclosed herein have high myocardial differentiation potential. Pluripotent stem cells are defined to have "high myocardial differentiation potential" when the cell population obtained by inducing myocardial differentiation of the pluripotent stem cells has at least one of the following features; (1) high beating rate, preferably about 70% or more, more preferably about 75% or more, still more preferably about 80% or more, wherein the beating rate is the percentage of beating cell clusters in the total cell clusters; (2) strong beating, preferably strain rate (1/s) of about 0.02, about 0.03, about 0.04, about 0.05 or more; (3) high percentage of cTnT positive cells, preferably about 35%, about 40%, about 45%, about 50% or more; and (4) high expression level of a cardiomyocyte-specific marker.

Examples of the cardiomyocyte-specific markers include $\alpha$-MHC, $\beta$-MHC, ACTN2, MYH7, SLC8A1, MYL2, TNNI3, CKM, MYL3, TNNT2, DES, MYL7, NAT1, GATA4, NKX2-5, HAND2, NPPA, KCNQ1, PLN, MB, RYR2, and PPC. Expression of a cardiomyocyte-specific marker may be detected by a conventional biochemical or immunochemical method, e.g., an enzyme-linked immunosorbent assay or an immunohistochemical assay. Alternatively, expression of a nucleic acid encoding a cardiomyocyte-specific marker may be evaluated. In one embodiment, a cardiomyocyte population obtained by inducing myocardial differentiation of DCoG cells expresses *MYH7* gene, which encodes $\beta$-MHC, at a level about four times higher than a cardiomyocyte population obtained by inducing myocardial differentiation of MCoG cells.

In an aspect, the disclosure provides a composition for preparing a cardiomyocyte comprising a pluripotent stem cell prepared by the method described above. The composition contains such pluripotent stem cell in a medium or solution suitable for survival, proliferation, or storage thereof. Such a medium or solution is known to those skilled in the art and may be readily prepared or commercially available. The pluripotent stem cell may or may not be frozen.

In an aspect, the disclosure provides a method of preparing a population of cardiomyocytes comprising inducing myocardial differentiation of a population of pluripotent stem cells prepared by the method described above. As demonstrated by the Examples below, especially Result (2), when myocardial differentiation of DCoG cells and MCoG cells is induced, DCoG cells differentiate into more highly matured cardiomyocytes with higher efficiency than MCoG cells. Conventionally, DCoG cells and MCoG cells have not been distinguished from each other and myocardial differentiation of a mixture of DCoG cells and MCoG cells has been induced, resulting in a cardiomyocyte population comprising cardiomyocytes having different maturation levels. A population of cardiomyocytes obtained by inducing myocardial differentiation of DCoG cells is more highly matured and more homogeneous than a population of cardiomyocytes obtained by a conventional method.

A population of cardiomyocyte is defined to be "highly matured" when it has at least one of the following features; (1) high beating rate, preferably about 70% or more, more preferably about 75% or more, still more preferably about 80% or more, wherein the beating rate is the percentage of beating cell clusters in the total cell clusters; (2) strong beating, preferably strain rate (1/s) of about 0.02, about 0.03, about 0.04, about 0.05 or more; (3) high percentage of cTnT positive cells, preferably about 35%, about 40%, about 45%, about 50% or more; and (4) high expression level of a cardiomyocyte-specific marker.

Various methods are known for inducing myocardial differentiation of pluripotent stem cells and any one of such methods may be used herein. Examples of the known methods include the methods described in Patent Literatures 1 and 2 and Non-Patent Literatures 4 and 5. Any medium suitable for inducing myocardial differentiation, such as RPMI/B27 and IMDM, may be used. Myocardial differentiation may be induced on a low cell adhesion substrate or on any other suitable substrate.

In one embodiment, myocardial differentiation of pluripotent stem cells is induced by the method described in Non-Patent Literature 5. Briefly, the method comprises (1) culturing pluripotent stem cells in an insulin-free medium containing a GSK3 inhibitor or a WNT signaling activator, e.g., CHIR99021, and optionally a ROCK inhibitor, e.g., y27632; (2) culturing the cells obtained in step (1) in an insulin-free medium containing a WNT signaling inhibitor, e.g., a porcupine inhibitor such as IWP2 or IW4, a β-catenin inhibitor such as β-catenin shRNA, SO3042 (2-(4-(3,4-dimethoxyphenyl)butaneamide)-6-iodobenzothiazole, KY03-I), or XAV939; and (3) culturing the cells obtained in step (2) in a medium containing insulin.

In one embodiment, myocardial differentiation of pluripotent stem cells is induced by the method described in Patent Literature 2. Briefly, the method comprises (1) culturing pluripotent stem cells in a medium containing a GSK3 inhibitor or a WNT signaling activator, e.g., CHIR99021, and a PKC activator, e.g., phorbol 12-myristate 13-acetate; and (2) culturing the cells obtained in step (1) in a medium containing a WNT signaling inhibitor, e.g., SO3042 or XAV939, a Src inhibitor, e.g., A419259, and an EGF receptor inhibitor, e.g., AG1478.

In an aspect, the disclosure provides a population of cardiomyocytes prepared by the method described above. In another aspect, the disclosure provides a composition comprising a population of cardiomyocytes prepared by the method described above. The composition contains a population of cardiomyocytes in a medium or solution suitable for survival or storage of the cardiomyocytes. Such a medium or solution is known to those skilled in the art and may be readily prepared or commercially available. The population of cardiomyocytes may or may not be frozen.

For example, the disclosure provides the following embodiments.

[1] A method of preparing a pluripotent stem cell suitable for differentiating into a cardiomyocyte, comprising (1) culturing at least one pluripotent stem cell on a low cell adhesion substrate to generate a dome-like colony; and (2) collecting a cell from the dome-like colony.

[2] The method according to item 1, wherein the substrate comprises a hydrogel or nanofiber.

[3] A method of preparing a pluripotent stem cell suitable for differentiating into a cardiomyocyte, comprising

(1) culturing at least one pluripotent stem cell on a substrate comprising a hydrogel or nanofiber to generate a dome-like colony; and
(2) collecting a cell from the dome-like colony.

[4] The method according to item 2 or 3, wherein the substrate comprises a hydrogel.

[5] The method according to item 5, wherein the hydrogel is Matrigel, fibronectin, vitronectin, or laminin.

[6] The method according to item 4 or 5, wherein the hydrogel is Matrigel, fibronectin, or vitronectin.

[7] The method according to item 6, wherein the concentration of Matrigel, fibronectin, or vitronectin is in the range of about 0.01 to 1.0 $\mu$g/cm$^2$.

[8] The method according to item 6 or 7, wherein the concentration of Matrigel, fibronectin, or vitronectin is in the range of about 0.05 to 0.3 $\mu$g/cm$^2$.

[9] The method according to any one of items 6 to 8, wherein the concentration of Matrigel, fibronectin, or vitronectin is about 0.1 $\mu$g/cm$^2$.

[10] The method according to any one of items 5 to 9, wherein the hydrogel is Matrigel.

[11] The method according to item 5, wherein the hydrogel is laminin.

[12] The method according to item 11, wherein the concentration of laminin is in the range of about 0.01 to 0.1 $\mu$g/cm$^2$.

[13] The method according to item 11 or 12, wherein the concentration of laminin is about 0.05 $\mu$g/cm$^2$.

[14] The method according to item 2 or 3, wherein the substrate comprises a nanofiber.

[15] The method according to item 14, wherein the nanofiber comprises gelatin, collagen, polyglycolic acid (PGA), polylactic acid (PLA) or copolymer thereof (PLGA), polycaprolactone (PCL), or polystyrene (PS).

[16] The method according to item 14 or 15, wherein the nanofiber is a gelatin nanofiber.

[17] The method according to item 16, wherein the concentration of the gelatin nanofiber is in the range of about 3 to 5 $\mu$g/cm$^2$.

[18] The method according to item 16 or 17, wherein the diameter of the gelatin nanofiber is in the range of about 50 to 500 nm.

[19] The method according to any one of items 16 to 18, wherein the gelatin nanofiber comprises gelatin having a molecular weight in the range of about 30 to 40 kDa.

[20] The method according to any one of items 16 to 19, wherein the gelatin nanofiber is cross-linked.

[21] The method according to any one of items 1 to 20, wherein the height of the dome-like colony is 50 $\mu$m or more, 60 $\mu$m or more, 70 $\mu$m or more, 80 $\mu$m or more, 90 $\mu$m or more, or 100 $\mu$m or more.

[22] The method according to any one of items 1 to 21, wherein the height of the dome-like colony is 50 $\mu$m or more.

[23] The method according to any one of items 1 to 22, wherein the dome-like colony generated in step (1) is derived from one pluripotent stem cell.

[24] A composition for preparing a cardiomyocyte, comprising a pluripotent stem cell prepared by the method according to any one of items 1 to 23.

[25] A method of producing the composition according to item 22, comprising the method according to any one of items 1 to 23.

[26] A method of preparing a population of cardiomyocytes, comprising inducing myocardial differentiation of a population of pluripotent stem cells prepared according to any one of items 1 to 23.

[27] A method of preparing a population of cardiomyocytes, comprising

(1) preparing a population of pluripotent stem cells by the method according to any one of items 1 to 23; and
(2) inducing myocardial differentiation of the population obtained in step (1).

[26] A population of cardiomyocytes prepared by the method according to item 26 or 27.

The entire contents of the documents cited herein are incorporated herein by reference.

The embodiments described above are non-limiting and may be modified without deviating from the scope of the invention as defined by the appended claims. The following example does not restrict or limit the invention and is for illustrative purposes only.

EXAMPLES

Materials and methods

Single-cell culture device fabrication

A gelatin nanofibrous (GNF) substrate was generated by electrospinning (the distance between the nozzle and the collector, i.e., a cover glass slide: 11 cm, syringe needle: 23 G, voltage: 11 kV, flow rate: 0.2 mL/hr) on culture cover glass slides.

Gelatin (11 wt%, type B, from porcine skin; Sigma-Aldrich, USA) solutions were prepared by dissolving gelatin in a mixture of acetic acid (42 wt%), ethyl acetate (28 wt%), and distilled water (20 wt%) for 16 hr prior to electrospinning. To obtain GNF samples with different concentrations, the electrospinning time was 8 min. After electrospinning, the GNFs were cross-linked in 0.2 M N-ethyl-N'-(dimethylaminopropyl) carbodiimide and 0.2 M N-hydroxysuccinimide in ethanol for 4 hr. Before use, the GNFs were rinsed with 70% ethanol three times and dried.

A multi-well membrane was produced by spin-coating polydimethylsiloxane (PDMS, Sylgard 184 from Dow Corning Toray, Japan) at a ratio of 1:10 on a silicon wafer containing an array of 100-$\mu$m-tall pillars. The mold was obtained by standard photolithography with SU8-100 (Microchem, Japan). After curing at 70°C for 10 min, the PDMS membrane was peeled from the mold. After rinsing with ethanol and drying, the micro-well was placed on the GNF samples. This gave a single-cell culture device having 400 wells with a diameter of 1000 $\mu$m.

Single human pluripotent stem cell (hPSC) isolation and culture

Cells were collected from the GNF substrate and then counted and diluted to establish a culture with a concentration of $2 \times 10^3$ cells/mL. A cell suspension (200 mL) was seeded on the single-cell culture device. After 2 hr, 2 mL of conditioned medium, the culture supernatant prepared by culturing cells of the same type in mTeSR-1 medium on a GNF substrate for 1-3 days, was added and changed every 1-2 days. After formation of the single cell-derived colonies, they were dissociated with an enzyme-free solution and re-seeded on a new GNF substrate for proliferation.

Pluripotent stem cell culture

The human iPSC lines 253G1 and IMR, and the human ESC lines H1 and H9 were used for this study. hESCs were used following the Kyoto University guidelines. Cells were seeded at $4 \times 10^4$ cells/cm$^2$ in mTeSR-1 cell culture medium (Stem Cell Technologies, USA) supplemented with 10 $\mu$M of the ROCK inhibitor Y27632 (Wako Chemicals, Japan) on GNF or Matrigel (MG)-coated substrates (BD Biosciences, USA). After 48 hr, the medium was changed to medium without Y27632. The culture medium was changed daily. Cells were dissociated to single cells and passaged every 3-4 days. Non-enzymatic cell dissociation ethylenediaminetetraacetic acid-based solution (Thermo Fisher Scientific, USA) was used to harvest cells cultured on the GNF substrate, and TrypLE Express (GIBCO, USA) was used for dissociating cells cultured on the MG-coated substrate. For obtaining a close comparison, two types of cells were cultured with exactly the same culture condition. The images of different areas were captured by continuous scanning with a 20-min interval for 48 hr under a microscope (IX81, Olympus Co., Japan) at 37°C and 5% $CO_2$.

Results

(1) Characterization of pluripotent stem cells cultured on GNF substrate

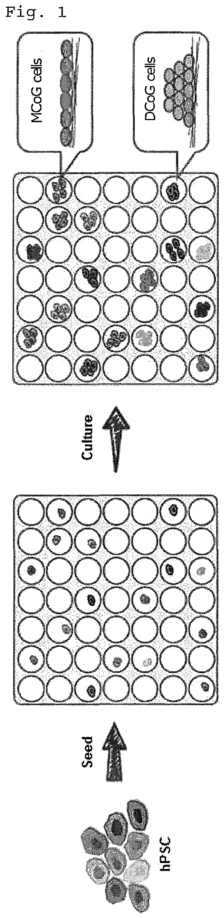

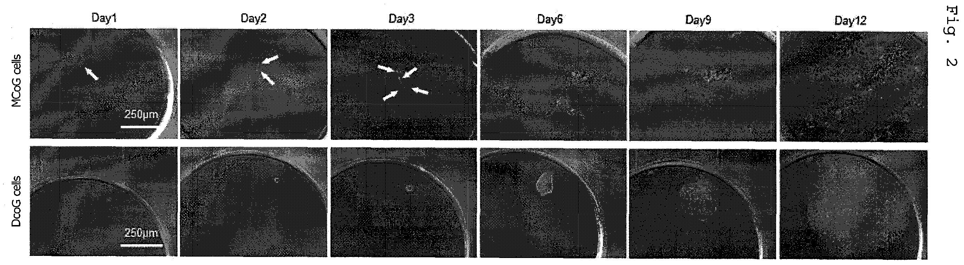

Two subtypes of colonies which differ in their morphology were observed when human iPS cells (hiPSCs) were cultured on the GNF substrate, unlike on the conventional MG-coated substrate, under the same hiPSC culture condition. To better investigate this phenomenon, a device for single-cell culture was developed, which comprised a polydimethylsiloxane multi-well membrane and a GNF substrate (Fig. 1). Single hPSCs were isolated and cultured by using a cocktail conditioning medium, which again grew into two types of morphologically distinguishable clones (Fig. 2).

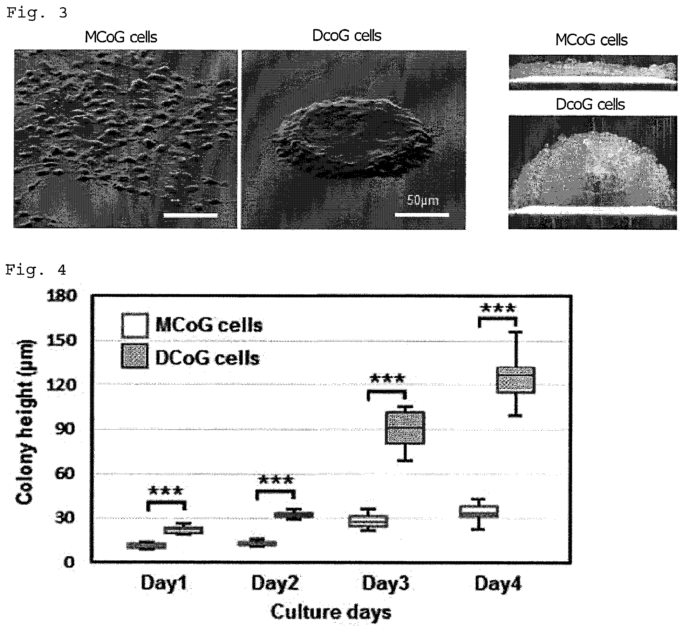

The existence of these two subtypes was confirmed in different hPSC lines (hiPSC line: 253G1 and IMR, human ES cell (hESC) line: H1 and H9). When grown on the GNF substrate, the majority of single cell-derived clones showed a flat monolayer colony morphology, designated as a monolayer colony on Gelatin (MCoG), and the other subtype showed a compact dome-like colony morphology, designated as a dome-like colony on Gelatin (DCoG) (Fig. 3). The three-dimensional scanning result indicated that the DCoG-type cells demonstrated a multilayer structure, in comparison with the monolayer structure of the MCoG-type cells. The colony height was also greatly different between them (Fig. 4). Real-time observation of MCoG and DCoG cells revealed that the two types of cells demonstrated different morphologies after attachment, which later became more and more distinct during proliferation. In addition, the self-renewal property and morphology of these single-cell-derived clones could be stably maintained on the GNF substrate for more than 27 passages. When another isolation of single cells from these two subtypes of cells was performed, the resulting clones maintained their respective morphologies. Short tandem repeat analysis has obviated the possibility of the cross-contamination between cell lines.

The average doubling time of DCoG cells was $22 \pm 0.7$ hr, which was shorter than that of MCoG cells at $26.5 \pm 1.5$ hr. Flow cytometric-mediated cell-cycle analysis showed a larger percentage (71.55%) of DCoG cells in the M/G2 and S phases compared with MCoG cells (58.43%) (in MCoG cells, G0/G1 phase: 38.53%, S phase: 28.26%, M/G2 phase: 30.17%, in DCoG cells, G0/G1 phase: 27.53%, S phase: 25.46%, M/G2 phase: 46.09%).

The pluripotency of these two subtypes after 26 passages was evaluated. Both subtypes were positive for pluripotency markers such as OCT4, NANOG, and alkaline phosphatase, with minimal levels of lineage commitment markers *(PAX6, Brachyury* and *AFP)*. Flow cytometric analysis showed that over 99.6% of the cells expressed SSEA4 and TRA-1-60. Next, the differentiation potential of the two subtypes was investigated both *in vitro* and *in vivo.* MCoG and DCoG cells formed embryonic bodies (EBs) and teratomas, which were able to differentiate into cells of all three germ layers. Furthermore, these clones still maintained a normal karyotype for over 27 passages. These results indicate that both subtypes derived from single hPSCs are able to maintain proper pluripotent status after long-term culture on the GNF substrate.

(2) Myocardial differentiation potential of DCoG cells

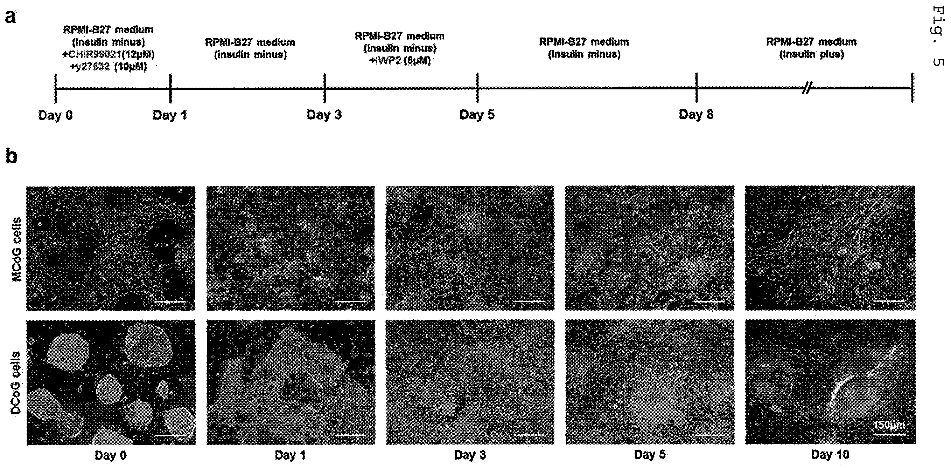

To investigate biological differences between the subtypes, myocardial differentiation of the cells were induced by modulating Wnt signaling in the manner described in Patent Literature 5 (Fig. 5). Briefly, MCoG and DCoG cells were cultured in an insulin-free RPMI/B27 medium containing CHIR99021 (12 $\mu$M) and y27632 (10 $\mu$M) for two days, in an insulin-free RPMI/B27 medium for two days, in an insulin-free RPMI/B27 medium containing IWP2 (5 $\mu$M) for two days, and in an insulin-free RPMI/B27 medium for three days, and then the cells were maintained in an RPMI/B27 medium containing insulin.

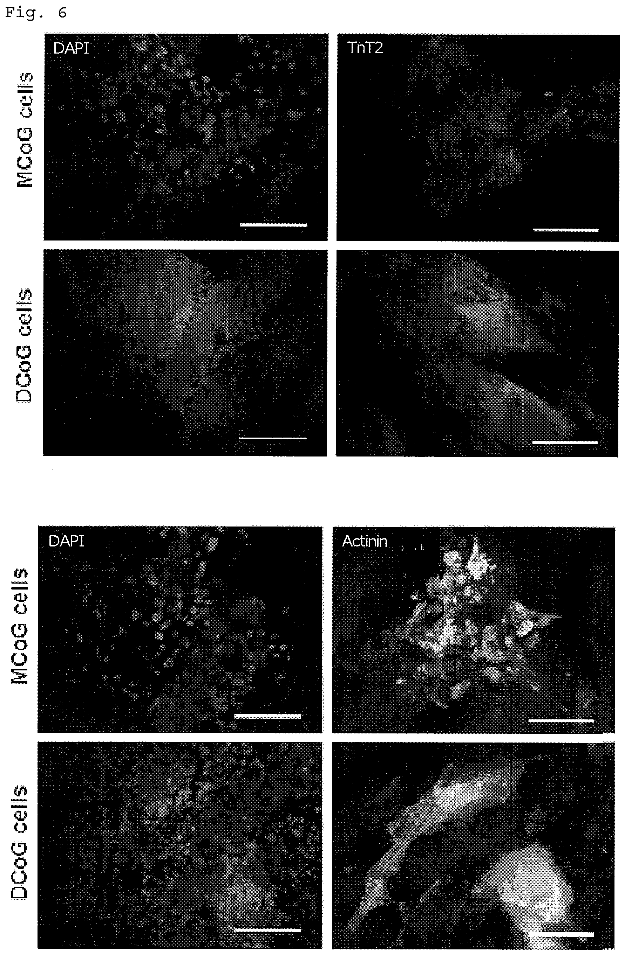

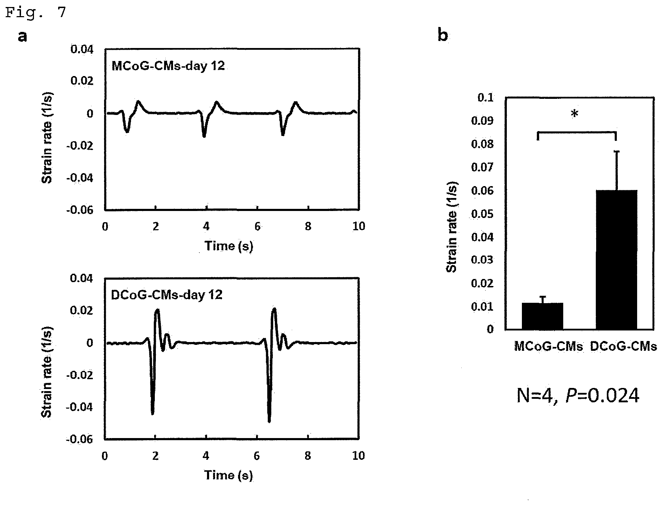

On Day 9, the percentage of beating clusters in total clusters was 69% and 84% for MCoG group and DCoG group, respectively. On Day 10, the flow cytometric analysis indicated that cells which are positive for cTnT, a cardiomyocytes-specific marker, accounted for $31.9 \pm 9.3\%$ in MCoG group and for $56.4 \pm 14.6\%$ in DCoG group. Besides the significant difference in the differentiation efficiency, the differentiated cardiomyocyte tissue structure also showed difference between MCoG and DCoG groups. An elongated structure similar to the cardiomyocyte structure in a living body was observed in DCoG group, but not in MCoG group (Fig. 6). Strain maps indicating contraction functions of the cardiomyocytes were created by recording the movement of the cardiomyocytes and tracking pixel movements in a frame to frame cross correlation algorithm (Eng, G. et al., Nat. Commun. 7, 1-10 (2016)). The cardiomyocytes derived from DCoG cells exhibited a higher strain rate, which indicates the amount of deformation of cells with respect to time, than those derived from MCoG cells (Fig. 7a). The strain per beating was 6 times higher in DCoG group than in MCoG group (Fig. 7b).

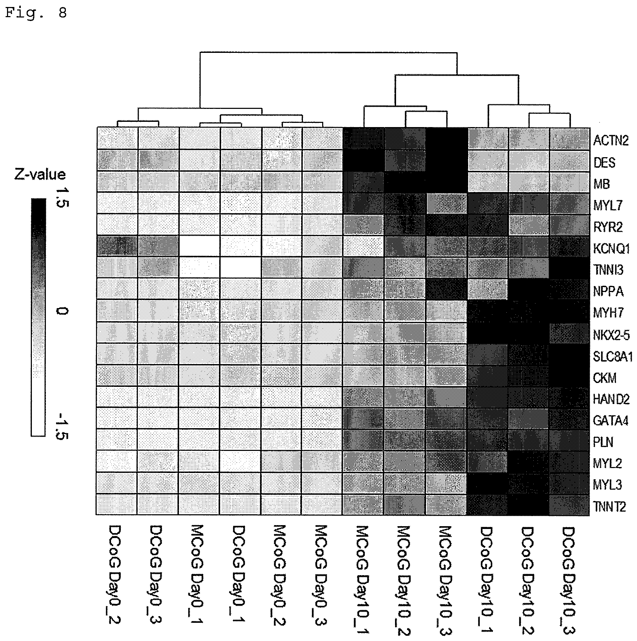

Next investigated was the expression of genes related to cardiomyocytes in the two subtypes. A number of genes were upregulated in DCoG group compared with MCoG group, which include the genes involved in ventricular structures *(MYL2, HAND2, MYL3),* cardiac sodium/potassium channel (*SLC8A1, KCNQ1*), early cardiac transcription factors (GATA4 and *NKX2-5*), ER-Ca2+ function (*PLN*), β1-adrenoceptor (*ADRB1*), atrial natriuretic peptide (*NPPA*), and ATP activities (*CKM*) (Fig. 8). Moreover, in DCoG cells the expression level of *MYH7* gene, which encodes β-MHC, an important marker for cardiomyocytes maturation, was 4 times higher than that in MCoG cells.

These results indicate that the cardiomyocytes derived from DCoG cells are more matured and functional than those derived from MCoG cells, and the differentiation efficiency is higher in DCoG cells. It has been reported that differentiation efficiencies vary among different stem cell lines. The results demonstrate that differentiation efficiencies also vary among different subtypes within the same cell line, implying that more appropriate precursors should be selected for inducing differentiation of PSCs.

(3) Substrate effect on MCoG and DCoG cells

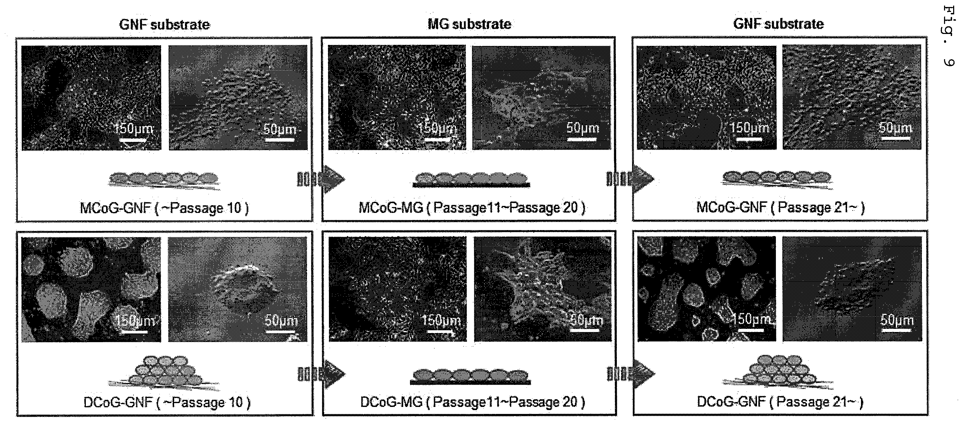

To investigate how the substrate effects on the two cell subtypes, the GNF substrate was replaced with a MG substrate (MG concentration: 10 $\mu$g/cm$^2$) over 10 passages. Plating DCoG cells on MG resulted in a morphological change from domed to a flat monolayer. Interestingly, these cells formed domed colonies again when re-plated onto the GNF substrate. In contrast, the colony morphology of MCoG cells remained unchanged when plated on either the GNF or MG substrate (Fig. 9). The result supports the notion that DCoG-type cells are sensitive to varying adhesion of substrates, but that MCoG-type cells are not, indicating some intrinsic differences between the two cell subtypes, which were concealed on the conventional substrates. Typically, DCoG cells are substrate-sensitive, and MCoG cells are substrate-insensitive. The majority of standard PSCs are the substrate-insensitive cells, which are not sensitive to variation of substrate properties. In this study, >90% of the isolated single cells were substrate-insensitive.

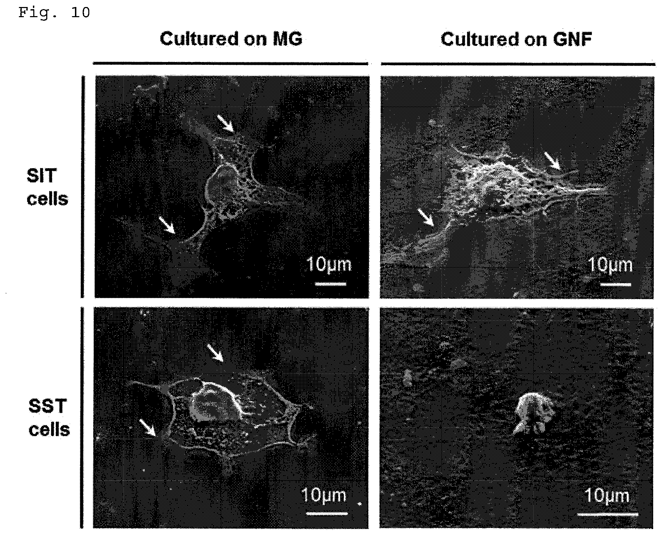

The substrate effect on MCoG and DCoG cells at the single-cell level was observed (Fig. 10). DCoG cells grown on the GNF substrate, without spreading, formed very few and short filopodia, and were hemispherical. By contrast, MCoG cells were flat and spread, and formed long filopodia on both the GNF and MG substrates. DCoG cells were similar to MCoG cells when plated on the MG substrate, where they spread well and formed long filopodia.

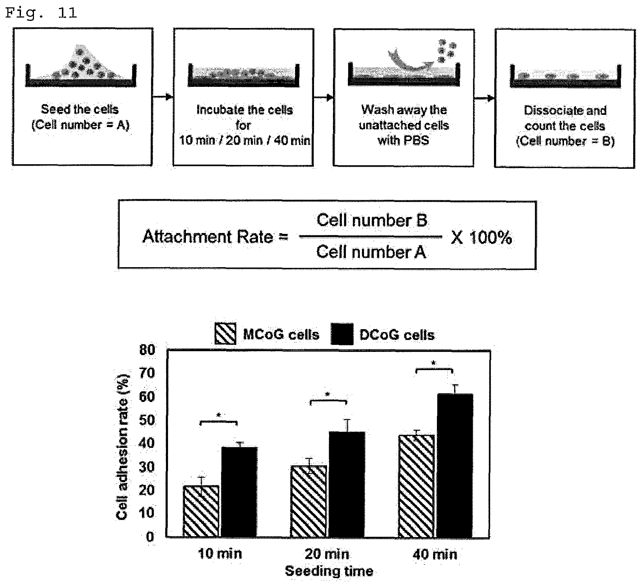

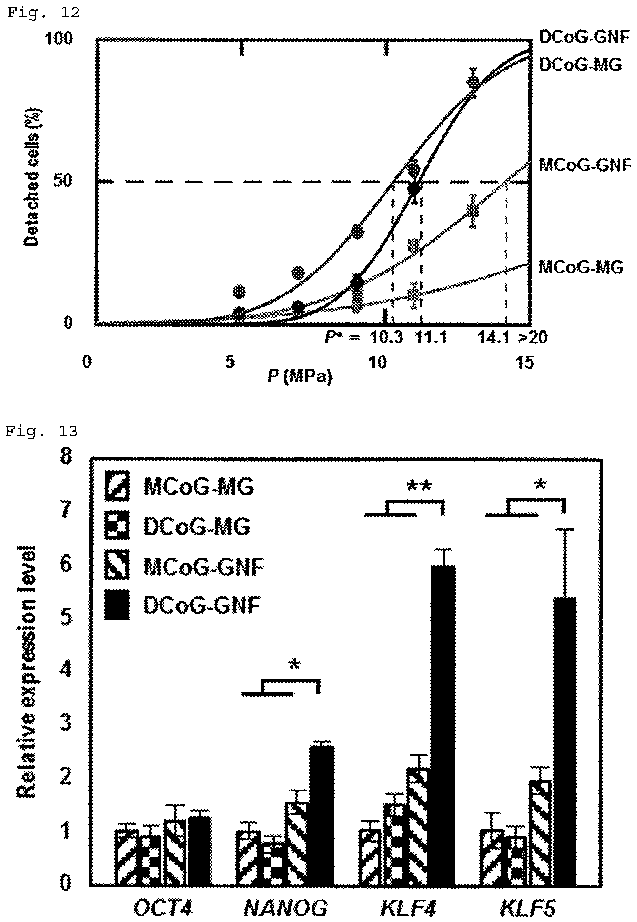

The RNA sequencing result showed different expression patterns in 3092 genes between MCoG cells and DCoG cells, and functional annotation analysis with gene ontology (GO) clearly revealed that the downregulated genes in DCoG cells were mostly related to adhesion, and indicated low cell adhesion of DCoG cells compared with McoG cells, which was also confirmed with a cell adhesion test (Fig. 11). On the other hand, previous study indicated that the GNF substrate has a lower adhesion property than conventional substrates, e.g., a substrate coated with MG or laminin (LA) at a concentration lower than that recommended by the manufacturer (Non-patent literature 5). Here the cell-substrate adhesion was quantified by using a shock wave based methods (Yoshikawa, H. Y. et al., J. Am. Chem. Soc. 133, 1367-1374 (2011)). Among all four conditions tested (DCoG and MCoG cells on GNF and MG substrates), the DCoG cells on the GNF substrate showed the lowest cell-matrix adhesion (Fig. 12), and it should be noted that only DCoG cells on GNF substrate formed domed colonies. The expression of the hPSC-specific genes was examined in these four conditions and it was found that the DCoG type cells cultured on GNF showed higher expression levels of *NANOG* and *KLF4*/*KLF5* compared with other conditions (Fig. 13).

To investigate the mechanism underlying these effects, serum response factor (SRF) was focused on. SRF is a transcription factor that regulates cell adhesion, motility, morphology and fate decisions, and activated only when interacting with its co-factor MAL, also known as MRTF-A or MKL1, which is sensitive to variations of global-actin (G-actin) levels. In the nucleus, G-actin binds to MAL, preventing it from binding to SRF and activating G-actin-dependent nuclear export to reduce the amount of MAL in the nucleus, resulting in the suppression of SRF transcription. G-actin exists as a monomeric form of the filamentous actin (F-actin) cytoskeleton, both of which can be interconverted. It has been reported that levels of F-actin may increase with the increase of adhesion and spreading of cells. On the other hand, depolymerization of F-actin can promote the reprogramming process, and thus enhances iPSC generation.

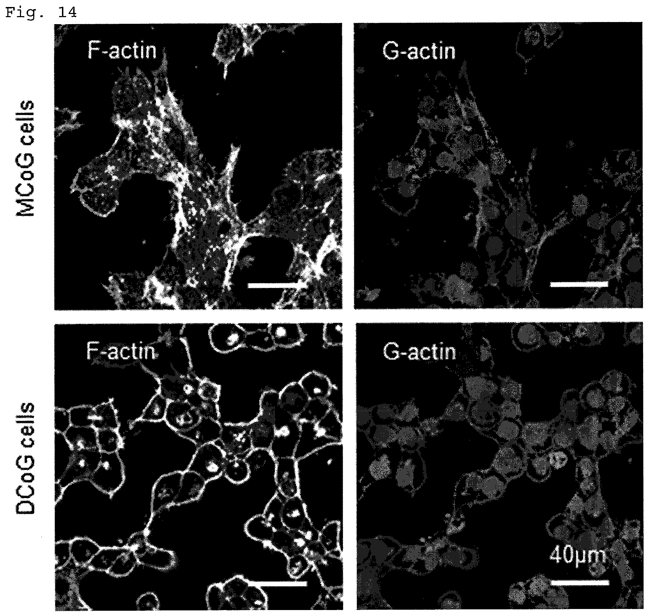

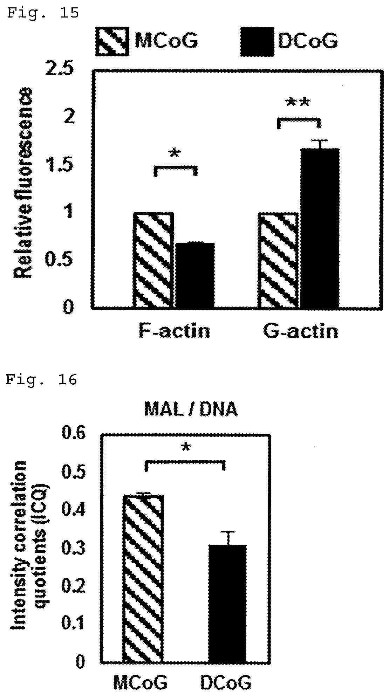

When cells were grown on the low-adhesion GNF substrate, the distinct F-actin stress fibers spread extensively in the cell filopodia and body of MCoG cells. In contrast, F-actin was arranged in a cortical cell shell of DCoG cells (Fig. 14). Quantification of phalloidin and DNase I with fluorescence analysis indicated a significantly lower level of F-actin and higher level of G-actin in DCoG cells (Fig. 15). The increased level of G-actin resulted in greater export of MAL from the nucleus to the cytoplasm in DCoG cells. In comparison, MAL was concentrated in the nucleus of MCoG cells (Fig. 16). In addition, on the high-adhesion MG substrate there were no differences in the actin cytoskeleton and MAL distribution between DCoG and MCoG cells.

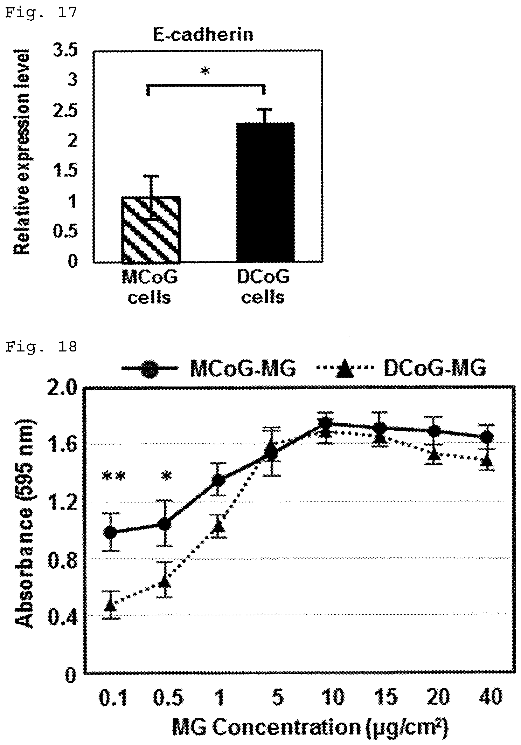

The appearance of the two types of cells suggests that there may be substantial differences in the density of cell-cell contacts. The higher E-cadherin expression indicated a strong cell-cell interaction in DCoG cells compared with MCoG cells (Fig. 17).

(4) DCoG and MCoG cells cultured on different substrates

Cell-matrix adhesion was determined as described in Miyazaki, T. et al., Nat. Commun. 3, 1210-1236 (2012). Substrates having different adhesion properties were prepared by varying coating concentration of hydrogels (Matrigel, fibronectin, vitronectin, and laminin). On substrates coated with the recommended concentration of hydrogels, e.g.

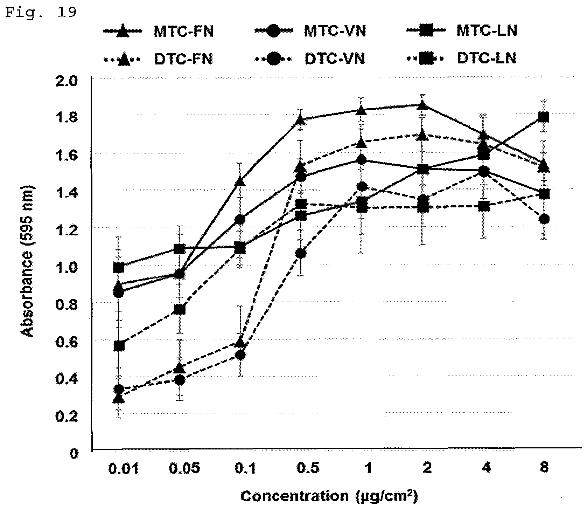

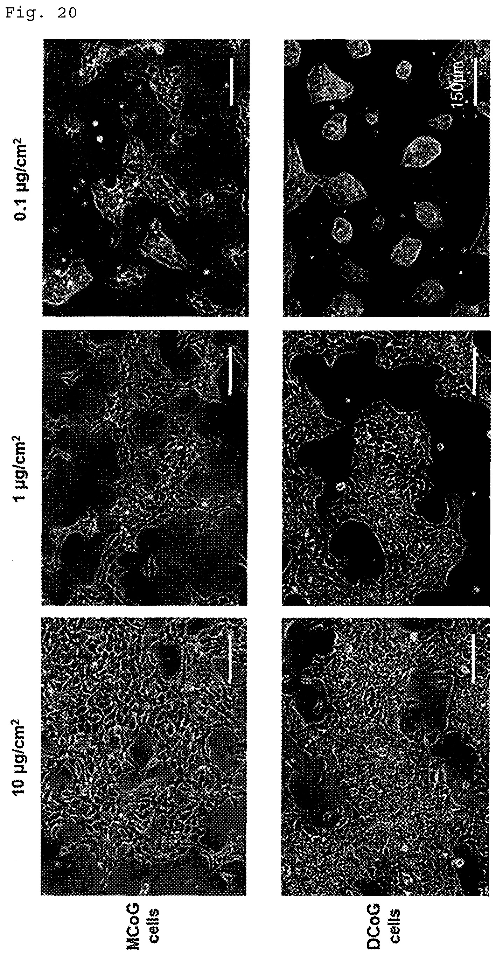

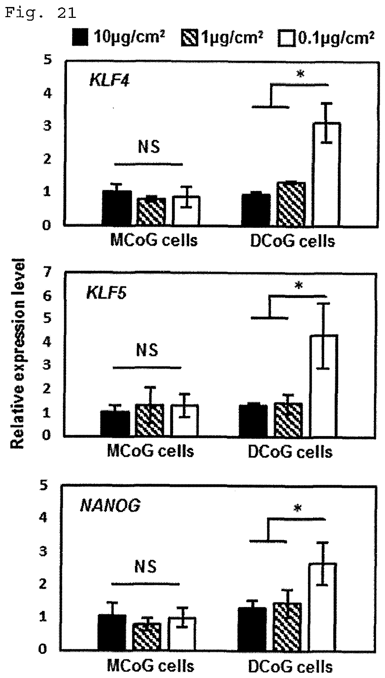

Matrigel of 10 $\mu$g/cm$^2$, the two types of cells showed similar cell-matrix adhesion. However, the cell-matrix adhesion of DCoG cells dropped more rapidly than MCoG cells with decreasing coating concentration (Fig. 18). The similar phenomenon was observed for the cells on fibronectin, vitronectin, and laminin (Fig. 19). While DCoG cells cultured on sparsely MG-coated substrates (0.1 $\mu$g/cm$^2$) demonstrated domed morphology and upregulation of *KLF4/5* and *NANOG* expression, on densely MG-coated substrates (1 and 10 $\mu$g/cm$^2$) they showed monolayer morphology and an expression level of *KLF4/5* and *NANOG* genes similar to MCoG cells (Figs. 20 and 21). In contrast, MCoG cells exhibited no obvious changes in the morphology and the expression pattern with varying MG coating concentration (Figs. 20 and 21). These results revealed that when cultured on sparsely MG-coated substrates DCoG cells demonstrate domed morphology similar to that observed on the GNF substrate. Together with the results of cell-matrix adhesion measurements, DCoG cells are considered to demonstrate domed morphology when cultured on substrates sparsely coated with other hydrogels.

INDUSTRIAL APPLICABILITY

The disclosure is useful for preparing a population of highly matured cardiomyocytes. For example, a population of highly matured cardiomyocytes is expected to contribute to regenerative therapy and drug evaluation in view of effects and toxicities, especially screening of drugs for a cardiac disease or evaluation of cardiotoxicity of drug candidates.

**Claims**

1. A method of preparing a pluripotent stem cell suitable for differentiating into a cardiomyocyte, comprising

    (1) culturing at least one pluripotent stem cell on a low cell adhesion substrate to generate a dome-like colony; and
    (2) collecting a cell from the dome-like colony.

2. The method according to claim 1, wherein the substrate comprises a hydrogel or nanofiber.

3. The method according to claim 2, wherein the substrate comprises a hydrogel selected from Matrigel, fibronectin, vitronectin, and laminin.

4. The method according to any one of claims 1 to 3, wherein the substrate comprises a hydrogel selected from Matrigel having a concentration in the range of about 0.01 to 1.0 $\mu$g/cm$^2$, fibronectin having a concentration in the range of about 0.01 to 1.0 $\mu$g/cm$^2$, vitronectin having a concentration in the range of about 0.01 to 1.0 $\mu$g/cm$^2$, and laminin having a concentration in the range of about 0.01 to 0.1 $\mu$g/cm$^2$.

5. The method according to claim 1 or 2, wherein the substrate comprises a gelatin nanofiber.

6. The method according to claim 5, wherein the concentration of the gelatin nanofiber is in the range of about 3 to 5 $\mu$g/cm$^2$.

7. The method according to any one of claims 1 to 6, wherein the height of the dome-like colony is 50 $\mu$m or more.

8. The method according to any one of claims 1 to 7, wherein the dome-like colony generated in step (1) is derived from one pluripotent stem cell.

9. A composition for preparing a cardiomyocyte, comprising a pluripotent stem cell prepared by the method according to any one of claims 1 to 8.

10. A method of preparing a population of cardiomyocytes, comprising inducing myocardial differentiation of a population of pluripotent stem cells prepared by the method according to any one of claims 1 to 8.

11. A population of cardiomyocytes prepared by the method according to claim 10.

Fig. 1

Fig. 2

Fig. 3

MCoG cells          DcoG cells          MCoG cells

DcoG cells

50µm

Fig. 4

Fig. 5

Fig. 6

Fig. 7

a

b

N=4, *P*=0.024

Fig. 8

Fig. 9

Fig. 10

Fig. 11

Attachment Rate = $\dfrac{\text{Cell number B}}{\text{Cell number A}}$ X 100%

Fig. 12

Fig. 13

Fig. 14

Fig. 15

Fig. 16

Fig. 17

Fig. 18

Fig. 19

Fig. 20

Fig. 21

## INTERNATIONAL SEARCH REPORT

| International application No. |
| --- |
| PCT/JP2018/038729 |

**A. CLASSIFICATION OF SUBJECT MATTER**
Int.Cl. C12N5/0735(2010.01)i, C12N5/077(2010.01)i, C12N5/10(2006.01)i

According to International Patent Classification (IPC) or to both national classification and IPC

**B. FIELDS SEARCHED**

Minimum documentation searched (classification system followed by classification symbols)
Int.Cl. C12N5/0735, C12N5/077, C12N5/10

| Documentation searched other than minimum documentation to the extent that such documents are included in the fields searched | |
| --- | --- |
| Published examined utility model applications of Japan | 1922–1996 |
| Published unexamined utility model applications of Japan | 1971–2018 |
| Registered utility model specifications of Japan | 1996–2018 |
| Published registered utility model applications of Japan | 1994–2018 |

Electronic data base consulted during the international search (name of data base and, where practicable, search terms used)
JSTPlus/JMEDPlus/JST7580 (JDreamIII)
CAplus/MEDLINE/EMBASE/BIOSIS/WPIDS (STN)

**C. DOCUMENTS CONSIDERED TO BE RELEVANT**

| Category* | Citation of document, with indication, where appropriate, of the relevant passages | Relevant to claim No. |
| --- | --- | --- |
| X<br>Y | TANG, Yadong et al., "Induction and differentiation of human induced pluripotent stem cells into functional cardiomyocytes on a compartmented monolayer of gelatin nanofibers", Nanoscale, 2016, vol. 8, pp. 14530–14540, abstract, fig. 1–8, pp. 14531–14532, 14536 | 1–2, 5–11<br>3–4 |

☒ Further documents are listed in the continuation of Box C.  ☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 14 December 2018 (14.12.2018) | 25 December 2018 (25.12.2018) |

| Name and mailing address of the ISA/ | Authorized officer |
| --- | --- |
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

**INTERNATIONAL SEARCH REPORT**

International application No.

PCT/JP2018/038729

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2015-501640 A (TSINGHUA UNIVERSITY) 19 January 2015, claims 1-5 & US 2014/0295553 A1, claims 1-5 & WO 2013/083073 A1 & EP 2796541 A1 & CN 103146572 A | 3-4 |
| Y | JP 2012-239444 A (ASAHI GLASS CO., LTD.) 10 December 2012, claims 1-5 (Family: none) | 3-4 |
| Y | JP 2015-195752 A (TOKYO WOMEN'S MEDICAL UNIVERSITY) 09 November 2015, claims 14-16 (Family: none) | 3-4 |
| X | WO 2006/093276 A1 (KYOTO UNIVERSITY) 08 September 2006, claims 1-19, paragraph [0008], examples & US 2008/0241111 A1, claims 1-19, paragraph [0008], examples & EP 1857544 A1 | 9-11 |
| A | JP 2013-247943 A (KYOTO UNIVERSITY) 12 December 2013, entire text (Family: none) | 1-11 |
| A | LIU, Li et al., "Nanofibrous gelatin substrates for long-term expansion of human pluripotent stem cells", Biomaterials, 2014, vol. 35, pp. 6259-6267 | 1-11 |
| P, X | YU, Leqian et al., "Low Cell-Matrix Adhesion Reveals Two Subtypes of Human Pluripotent Stem Cells", Stem Cell Reports, 10 July 2018, vol. 11, pp. 142-156 | 1-11 |

Form PCT/ISA/210 (continuation of second sheet) (January 2015)

**REFERENCES CITED IN THE DESCRIPTION**

**Patent documents cited in the description**

- JP 2017202029 A
- WO 2013111875 A
- WO 2015182765 A
- JP 6024047 B

**Non-patent literature cited in the description**

- **OSAFUNE, K. et al.** *Nat. Biotechnol.,* 2008, vol. 26, 2007-2009
- The International Stem Cell Initiative. *Nat. Biotechnol.,* 2007, vol. 25, 803-816
- **MINAMI I et al.** *Cell Rep.,* 29 November 2012, vol. 2 (5), 1448-60
- **LIAN, X. et al.** *Nat. Protoc.,* 2013, vol. 8, 162-175
- **L. LIU et al.** *Biomaterials,* 2014, vol. 35, 6259-67
- **YOSHIKAWA, H. Y. et al.** *J. Am. Chem. Soc.,* 2011, vol. 133, 1367-1374
- *Advanced Drug Delivery Reviews,* 2009, vol. 61 (12), 1084-1096
- *Journal of Cellular and Molecular Medicine,* 2009, vol. 13 (9B), 3475-3484
- **ENG, G. et al.** *Nat. Commun.,* 2016, vol. 7, 1-10
- **MIYAZAKI, T. et al.** *Nat. Commun.,* 2012, vol. 3, 1210-1236